# EUROPÄISCHE PATENTSCHRIFT

(11) **EP 3 968 836 B1**
(45) Veröffentlichungstag und Bekanntmachung des Hinweises auf die Patenterteilung: **31.08.2022**
(21) Anmeldenummer: 21725489.5
(22) Anmeldetag: 11.05.2021
(51) Int. Cl.: A61B 1/00, A61B 17/128, A61B 17/10, A61B 17/00, A61B 17/08, A61B 17/122, A61B 17/12

(54) **GEWEBECLIP-APPLIKATIONS-AUSRÜST- ODER NACHRÜSTSATZ**
TISSUE CLIP APPLICATION FITTING OR RETROFITTING SET
KIT D'ÉQUIPEMENT OU KIT D'ÉQUIPEMENT ULTÉRIEUR POUR L'APPLICATION DE CLIPS TISSULAIRES

(30) Priorität: 19.05.2020 DE 102020113535
(43) Veröffentlichungstag der Anmeldung: 23.03.2022
(73) Patentinhaber: Ovesco Endoscopy AG, 72076 Tübingen (DE)
(72) Erfinder: BRAUN, Markus, 71093 Weil im Schönbuch (DE)
(74) Vertreter: Winter, Brandl - Partnerschaft mbB
(86) Internationale Anmeldenummer: PCT/EP2021/062504
(87) Internationale Veröffentlichungsnummer: WO 2021/233740

(56) Entgegenhaltungen:
- WO-A1-97/45060
- US-A- 5 398 844
- US-A1- 2004 006 256

## Beschreibung

Die Erfindung betrifft einen Gewebeclip-Applikations-Ausrüst- oder Nachrüstsatz mit einem Kappenaufsatz, der dafür ausgebildet ist, an/auf den distalen Endabschnitt eines medizinischen Endoskops der Schaftbauart montiert bzw. aufgestülpt/aufgesetzt zu werden und der hierfür einen proximalen Montage-/Aufstülpabschnitt und einen distalen Gewebeclip-Halteabschnitt aufweist, in dessen Bereich innenseitig ein in Richtung distal offener Hohlraum ausgebildet ist und auf dessen einer, radial nach außen weisenden Umfangsfläche ein Gewebeclip radial abgestützt ist, welcher mittels einer, vorzugsweise vom proximalen Endabschnitt des Endoskops bzw. extrakorporal bedienbaren/betätigbaren, Abzieheinrichtung in Form eines auf der radialen Außenumfangsfläche des Gewebeclip-Halteabschnitts axialgleitend gelagerten und den Gewebeclip führenden/verlagernden/schiebenden Abziehrings in Richtung distal verlagerbar ist.

### Hintergrund der Erfindung

Endoskope sind im Allgemeinen medizinische Arbeitsgerätschaften zur visuellen Exploration und gegebenenfalls Manipulation von Hohlräumen in einem Patientenkörper. Zur Exploration weisen sie grundsätzlich optische Einrichtungen am distalen (patientenzugewandten bzw. benutzerabgewandten) Endoskopende (auch Endoskopkopf genannt) auf. Für die Manipulation weisen sie optional einen oder mehrere interne Arbeitskanäle auf, der/die sich ausgehend von einem proximalen (patientenabgewandten bzw. benutzerzugewandten) Endoskopabschnitt, welcher herkömmlicherweise aus dem Patientenhohlraum nach außen ragt, oder einem extrakorporalen Endoskopgriff durch einen sich daran anschließenden, flexiblen oder starren Endoskopschaft hindurch bis zum Endoskopkopf erstreckt und das extrakorporale Einführen bzw. Zuführen eines oder mehrerer medizinischer Instrumente, wie z.B. Zangen, Anker, Scheren, Nadeln, Schlingen, Messer und dgl., ermöglicht.

Derartige Endoskope können wahlweise mit zusätzlichen Fähigkeiten versehen werden, etwa indem am distalen Endoskopkopf eine Kappe oder Hülse auf den zumindest die Optik aufnehmenden/beinhaltenden Endoskopkopf radial außenseitig aufgesetzt wird. Dabei ist die Kappe oder Hülse mit bestimmten Funktionen versehen bzw. mit bestimmten Funktionselementen ausgerüstet. Dies ermöglicht es, dass das Endoskop neben seinen ursprünglichen Funktionen, nämlich der Exploration und/oder als Zugang für minimalinvasive (medizinische) Instrumente, auch als (minimalinvasives) Instrument selbst zur Ausführung eines interventionellen oder chirurgischen Vorgangs verwendet werden kann.

### Stand der Technik

Aus dem Stand der Technik sind bereits Endoskope mit zusätzlichen, mittels Kappen(-aufsätzen) realisierten Funktionen bekannt. So zeigt beispielsweise DE 10 2017 112 896 A1 einen Gewebeclip-Applikations-Ausrüst- bzw. Nachrüstsatz, dessen Kappenaufsatz auf das distale Ende eines Endoskops aufgesetzt werden kann, um mittels eines Abziehrings einen Gewebeclip von dem Kappensaufsatz abzustreifen.

Ein weiteres Beispiel einer Endoskopkappe ist in DE 20 2008 007 774 U1 offenbart. Die Endoskopkappe weist hierbei eine Halte- und Abzieheinrichtung für einen Gewebeclip, der auf eine Spreizhülse der Endoskopkappe aufschiebbar ist, auf. An der Vorderkante der Spreizhülse ist eine sich öffnende sowie die Kappenmantelwand beidseits aufschlitzende Stirnnut angeordnet. Weiter weist die Endoskopkappe einen Abziehfaden oder ein Gewebe auf, der/das in einem axial vorderen Kappenabschnitt die Stirnnut radial durchquert und an einer radialen Innenseite der Endoskopkappe verschiebbar in einen Endoskopkanal für dessen Betätigung eingeführt oder einführbar ist.

Auch in US 2013 / 0 325 039 A1 ist eine Endoskopkappe gezeigt, welche es erlaubt, einen Gewebeclip über eine proximal zu betätigende Abzieheinrichtung in Form eines Aktuatorkabels, welches mit einem innerhalb der Kappe angeordneten und den Gewebeclip verlagernden Träger-/Abziehring gekoppelt ist, abzuziehen. Ferner ist in US 5,398,844 A eine mit einem Endoskop koppelbare Vorrichtung offenbart, mit welcher mehrere Ligationsbänder nacheinander verwendet werden können. Die einzelnen Ligationsbänder werden dabei unabhängig voneinander über ihnen zugeordnete Abziehfäden betätigt.

Die im Stand der Technik offenbarten Endoskopkappen haben jedoch immer den Nachteil, dass innerhalb der Kappe geführte Abziehringe die nutzbare Querschnittsfläche des Arbeitskanals verkleinern. An der Außenumfangsfläche geführte und mit einem Ziehfaden betätigbare Abziehringe neigen hingegen zum Verkanten, was wiederum zu Beschädigungen führen kann.

### Zusammenfassung der Erfindung

Es sind die Aufgaben und Ziele der Erfindung, die Nachteile aus dem Stand der Technik zu beheben oder wenigstens zu mindern. Insbesondere soll ein Gewebeclip-Applikations-Ausrüst- oder Nachrüstsatz bereitgestellt werden, welcher das Risiko des Verkantens des Abziehrings beseitigt bzw. verringert, ohne die nutzbare Fläche des Arbeitskanals (übermäßig) einzuschränken.

Die Aufgaben und Ziele werden hinsichtlich eines gattungsgemäßen Gewebeclip-Applikations-Ausrüst- oder Nachrüstsatzes erfindungsgemäß durch den Gegenstand des Anspruchs 1 gelöst. Die Erfindung beruht also auf der Erkenntnis einer möglichst gleichmäßigen bzw. symmetrischen Krafteinleitung in den Abziehring.

Der Gewebeclip-Applikations-Ausrüst- oder Nachrüstsatz ist demgemäß erfindungsgemäß konfiguriert/dafür angepasst, dass bei Betätigung der Abzieheinrichtung eine über den Umfang des Abziehrings verteilte bzw. am Abziehring möglichst symmetrisch eingeleitete Axialkraft wirkt.

Hierfür wird gemäß einer ersten, ggf. unabhängig zu beanspruchenden Ausführung der vorliegenden Offenbarung konstruktiv das Prinzip eines einfachen Flaschenzugs angewendet, wonach
- ein (erstes) Seilzugende ortsfest (distal) gehalten ist,
- ein Mittenabschnitt des Seilzugs längsverschieblich mit der (proximalen) Last, im Konkreten dem Abziehring, gekoppelt ist und
- ein anderes (zweites) Seilzugende um eine distale Umlenkung herum in Richtung (proximaler) Last und ggf. darüber hinaus (in Richtung proximal) zurückgeführt ist.

Der mit der Last, nämlich dem Abziehring, wirkverbundene Mittenabschnitt kann somit so dimensioniert und/oder positioniert werden, dass dieser eine im Wesentlichen symmetrische Krafteinleitung auf die Last (Abziehring) bewirkt, wodurch das Verkannten vermieden wird. Im Konkreten kann der Mittenabschnitt des Seilzugs an wenigstens zwei in Umfangsrichtung des Endoskops/der (separaten) Endoskopkappe voneinander beabstandete (vorzugsweise diametral sich gegenüberliegende) Anlenkstellen (Einschlaufungen, Angriffspunkte) mit der Last (dem Abziehring) in (Gleit- )Kontakt stehen, sodass beim Ziehen des Seilzugs dessen Mittenabschnitt an den Anlenkstellen längsgleitet und dabei den Abziehring in Richtung distal nach dem Flaschenzugprinzip verschiebt. Vorzugsweise ist der Seitzugabschnitt zwischen den zumindest zwei Anlenkstellen am Abziehring in Umfangsrichtung längsgeführt (vorzugsweise an dessen proximaler Stirnseite), wodurch eine noch gleichmäßigere Krafteinleitung in den Abziehring über den entsprechenden Umfangsabschnitt möglich ist.

Vorteilhafte Ausführungsformen sind in den Unteransprüchen beansprucht und werden nachfolgend erläutert.

In einer bevorzugten Variante kann bei Bedienung/Betätigung der Abzieheinrichtung, wie vorstehend bereits angedeutet, an zumindest zwei über den Umfang, vorzugsweise gleichmäßig, verteilten Anlenkstellen/Angriffspunkten die Axialkraft auf den Abziehring wirken. Dabei kann es von Vorteil sein, wenn an dem Abziehring ein Abziehfaden/Abziehgewebe/Abziehkabel gekoppelt (eingeschlauft) ist, welcher ausgebildet ist, um mit einem Endabschnitt von dem Hohlraum zu dem proximalen Endabschnitt des Endoskops geführt und mit dem anderen Endabschnitt an dem Gewebeclip-Halteabschnitt fixiert zu werden. Besonders bevorzugt kann der eine Abziehfaden so an dem Kappenaufsatz geführt sein, dass bei Betätigung des Abziehfadens dieser den Abziehring an den zwei Angriffspunkten in Richtung distal zieht, was es erlaubt, den Abziehring an den zumindest zwei Angriffspunkten mit nur einem einzigen (nicht gegabelten) Abziehfaden in Richtung distal zu ziehen und gleichzeitig eine gleichmäßigere Krafteinleitung und damit einhergehend ein geringeres Kippmoment zu gewährleisten.

Gemäß einer besonders vorteilhaften Weiterbildung kann der Abziehfaden
- vorzugsweise von dem proximalen Endabschnitt des Endoskops durch den internen (oder externen) Arbeitskanal des Endoskops in den Hohlraum,
- von dem Hohlraum (der separaten Endoskopkappe) aus über eine in dem Gewebeclip- Halteabschnitt ausgebildete, erste distale Radialdurchgangsöffnung nach radial außen,
- anschließend durch eine in dem Abziehring ausgebildete, erste Axialdurchgangsöffnung,
- dann entlang der Außenumfangsfläche der (separaten) Endoskopkappe und durch eine in dem Abziehring, vorzugsweise um 180° versetzt zu der ersten Axialdurchgangsöffnung, ausgebildete, zweite Axialdurchgangsöffnung sowie
- eine in dem Gewebeclip-Halteabschnitt, vorzugsweise um 180° versetzt zu der ersten Radialdurchgangsöffnung, ausgebildete, zweite distale Radialdurchgangsöffnung in den Hohlraum geführt sein, wo er an dem Gewebeclip-Halteabschnitt fixiert sein kann (Fixierungsstelle).

Vorzugsweise können an dem Abziehring zumindest zwei, den Angriffspunkten zugeordnete Abziehfäden angeordnet sein. Somit kann sichergestellt werden, dass an jedem Angriffspunkt im Wesentlichen dieselbe Axialkraft anliegt und kein Kippmoment auftritt.

Ferner ist es auch denkbar, dass der Abziehfaden so an dem Kappenaufsatz entlanggeführt sein kann, dass sich mehr als zwei Angriffspunkte ausbilden. Dabei kann der Abziehfaden durch weitere Radialdurchgangsöffnungen und Axialdurchgangsöffnungen geführt werden, um zusätzliche Angriffspunkte zu erhalten. Insbesondere kann zwischen den Angriffspunkten, wie bei einem Flaschenzug, jeweils distal eine Umlenkstelle angeordnet bzw. ausgebildet sein. In einer besonders vorteilhaften Ausgestaltung kann zur Ausbildung der Umlenkstelle der Abziehfaden über die Vorderkante nach innen in den Hohlraum, durch eine weitere, distale Radialdurchgangsöffnung wieder nach außen und dort zum nächsten proximalen Angriffspunkt am Abziehring geführt werden.

Gemäß einer zweiten alternativen, ggf. unabhängig beanspruchbaren Ausführung der vorliegenden Erfindung weist der Abziehring einen an dem Kappenaufsatz, vorzugsweise an dessen Gewebeclip-Halteabschnitt, befestigten/fixierten Stütz- /Lagerelement beispielsweise in Form eines Stützrings mit einer axialen oder radialen Außenseite, welche, eine Steigung in Richtung distal aufweist (z.B. eine keilförmige distale Stirnseite oder eine Spindel) und einen längs der Außenumfangsfläche des Gewebeclip-Halteabschnitts axialgleitend sowie drehbar gelagerten und (zumindest teilweise) distal von dem Stützelement/Stützring angeordneten Schiebering, welcher eine mit der Steigung des Stützrings in Wirkeingriff stehende Profilierung aufweist.

Ferner ist an dem Schiebering der Abziehfaden angeordnet, derart, dass dieser bei dessen (manueller) Betätigung den Schiebering um dessen Rotationsachse dreht und infolge des Wirkeingriffs seiner Profilierung mit der Steigung des Stützrings in Richtung distal verlagert.

Hierfür ist es von Vorteil, wenn an einer dem Schiebering zugewandten Stirnfläche des Stützrings und an einer dem Stützring zugewandten Stirnfläche des Schieberings jeweils zumindest eine Rampe/Nocke/Stützkeil ausgebildet ist, deren Keilrichtung gegenläufig zueinander sind, so dass sich der Stützring und der Schiebering bei Betätigung des Abziehfadens und entsprechender Drehung des Schieberings relativ zum Stützring axial voneinander beabstanden.

Gemäß einer alternativen Weiterbildung kann an dem Schiebering ein Innengewinde und an dem Stützring ein mit dem Innengewinde in Eingriff stehendes Außengewinde mit entsprechender Steigung ausgebildet ist, so dass sich der Stützring und der Schiebering bei Betätigung des Abziehfadens axial voneinander beabstanden. Selbstverständlich kann auch an dem Stützring ein Innengewinde und an dem Schiebering ein mit dem Innengewinde in Eingriff stehendes Außengewinde ausgebildet sein, so dass sich der Stützring und der Schiebering bei Betätigung des Abziehfadens axial voneinander beabstanden.

Ferner kann es von Vorteil sein, wenn der zumindest eine Abziehfaden in einer in dem Gewebeclip-Halteabschnitt ausgebildeten Axialnut in Richtung distal und/oder proximal geführt ist, so dass der Gewebeclip beim Abstreifen den in der Axialnut geführten Abziehfaden überfährt.

Erfindungsgemäß kann der zumindest eine Abziehfaden über einen separaten, vorzugsweise seitlich entlang des Endoskopschafts angeordneten, Längskanal hin zu dem proximalen Endabschnitt des Endoskops geführt sein. Jedoch ist es auch denkbar, dass der zumindest eine Abziehfaden in einem internen Arbeitskanal des Endoskops hin zu dem proximalen Endabschnitt des Endoskops geführt ist.

Für einen optimierten Gebrauch des Gewebeclip-Applikations-Ausrüst- oder Nachrüstsatz kann der Kappenaufsatz mit einer Überwurfhülse ausgebildet sein, welche fest, vorzugsweise stoffeinstückig, mit dem Kappenaufsatz verbunden ist und den auf den Gewebeclip-Halteabschnitt aufgezogenen Gewebeclip radial, zumindest abschnittsweise, umgibt.

### Kurzbeschreibung der Figuren

Die Erfindung wird nachfolgend anhand bevorzugter Ausführungsbeispiele mit Hilfe von Figuren näher erläutert. Es zeigen:
- Fig. 1: eine schematische Ansicht eines an einem Endoskop befestigten Gewebeclip-Applikations-Ausrüst- oder Nachrüstsatzes gemäß einem Ausführungsbeispiel,
- Fig. 2: eine Seitenansicht des Gewebeclip-Applikations-Ausrüst- oder Nachrüstsatzes gemäß dem Ausführungsbeispiel,
- Fig. 3: eine Längsschnittansicht des Gewebeclip-Applikations-Ausrüst- oder Nachrüstsatzes gemäß einer Modifikation des Ausführungsbeispiels, und
- Fig. 4-6: schematische Teilansichten einer Abzieheinrichtung gemäß einem weiteren,

ggf. unabhängig zu beanspruchenden Ausführungsbeispiel, welches nicht nicht durch die beanspruchte Erfindung abgedeckt ist.

Die Figuren sind schematischer Natur und dienen lediglich dem Verständnis der Erfindung. Gleiche Elemente sind mit denselben Bezugszeichen versehen. Die Merkmale der verschiedenen Ausführungsbeispiele können untereinander ausgetauscht werden.

### Detaillierte Beschreibung bevorzugter Ausführungsformen

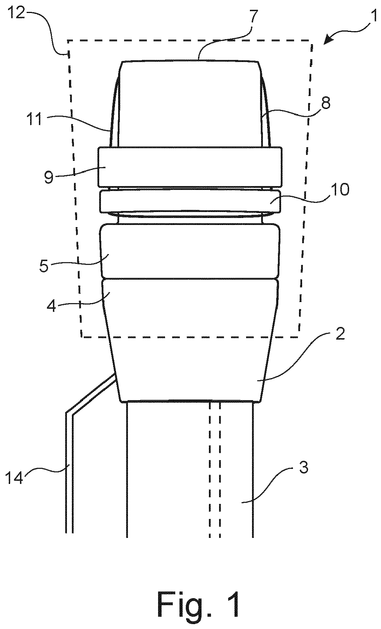

Fig. 1 zeigt eine schematische Ansicht eines Gewebeclip-Applikations-Ausrüst- oder Nachrüstsatzes 1, der als Kappenaufsatz 2 ausgebildet ist oder einen Kappenaufsatz 2 aufweist. Der Kappenaufsatz 2 kann dabei auf einen distalen Endabschnitt (Endoskopkopf) eines Endoskops 3 angesetzt, insbesondere aufgesetzt bzw. aufgestülpt werden. Das in Fig. 1 lediglich teilweise dargestellte Endoskop 3, wie es im Übrigen aus dem zuvor genannten Stand der Technik bekannt ist und dessen Beschreibung auch zum Gegenstand dieser Anmeldung gemacht wird, hat in der Regel einen (biegeflexiblen oder starren) Endoskopschaft, an dessen distalem Ende zumindest eine Optik und eine Beleuchtungseinrichtung eingebaut sind. Des Weiteren hat ein solches Endoskop 3 häufig einen internen Arbeitskanal, über den ein (minimalinvasives) medizinisches Instrument (z.B. eine Zange, Schere, und dergleichen) beispielsweise in ein Hohlorgan eines Patienten einführbar ist (in Fig. 1 gestrichelt dargestellt bzw. angedeutet). Schließlich kann am distalen Ende (Endoskopkopf) des Endoskops 3 noch eine Spülvorrichtung vorgesehen sein, mittels welcher die Optik gereinigt werden kann.

An seinem proximalen Endabschnitt weist das Endoskop 3 bekannter Bauart zudem einen Endoskopgriff auf, an welchem das Endoskop 3 gehalten und bedient werden kann. Dabei sei an dieser Stelle darauf hingewiesen, dass das Endoskop 3 anstatt eines Griffs auch eine Schnittstelle zu einer Robotik aufweisen kann.

Der Gewebeclip-Applikations-Ausrüst- oder Nachrüstsatz 1 bzw. der Kappenaufsatz 2 gemäß dem Ausführungsbeispiel weist einen proximalen Montage-/ Aufstülpabschnitt 4 und einen distalen Gewebeclip-Halteabschnitt 5 auf, wobei beide Abschnitte sich auch zumindest teilweise oder vollständig axial überlappen können. Wie vorstehend erwähnt, kann der Gewebeclip-Applikations-Ausrüst- oder Nachrüstsatz 1 mit seinem Montage-/ Aufstülpabschnitt 4 an dem distalen Endabschnitt des Endoskops 3 temporär montiert bzw. aufgestülpt werden. Der Montage-/ Aufstülpabschnitt 4 ist hierfür vorzugsweise in Form einer Muffe oder Manschette aus einem flexiblen, vorzugsweise elastischen, Material gebildet. Alternativ ist es aber auch möglich, den Montage-/ Aufstülpabschnitt 4 aus einer Kunststoffhülse zu fertigen, welche, z.B. durch Anordnung wenigstens eines Längsschlitzes oder infolge einer vorbestimmten Eigenflexibilität, radial aufweitbar ist. Ferner ist der Montage-/ Aufstülpabschnitt 4 dafür vorgesehen, auf den distalen Endabschnitt aufgestülpt zu werden, d.h. insbesondere nicht in den internen Arbeitskanal des Endoskops 3 eingeführt zu werden, um so quasi eine axiale Verlängerung des distalen Endabschnitts des Endoskopschafts zu bilden. Der interne Arbeitskanal des Endoskops 3 bleibt somit für das Einführen eines medizinischen Instruments offen und nutzbar.

An dieser Stelle sei erwähnt, dass der Kappenaufsatz 2 auch anderweitig mit dem Endoskopkopf des (handelsüblichen) Endoskops 3 gekoppelt werden kann, beispielsweise durch eine separate Überwurfhülse, einen Schrumpfschlauch und dergleichen Verbindungselemente.

Der Gewebeclip-Halteabschnitt 5 besteht aus einer gegenüber dem Montage-/ Aufstülpabschnitt 4 vorzugsweise starreren (hohlen) Kunststoffhülse, die vorzugsweise in Verlängerung zum Montage-/ Aufstülpabschnitt 4 einstückig, insbesondere stoffeinstückig, mit diesem verbunden oder mit dem Montage-/ Aufstülpabschnitt 4, beispielsweise durch Verkleben oder Verschweißen, gekoppelt ist. Zumindest der Gewebeclip-Halteabschnitt 5 bildet innenseitig eine Aushöhlung oder einen Hohlraum 6, der beispielsweise zur temporären Aufnahme von Patientengewebe vorgesehen ist und der am distalen Ende des Gewebeclip-Halteabschnitts 5 unter Ausbildung einer distalen Vorder- oder Stirnkante 7 in eine radial nach außen weisende Mantelfläche/ Außenumfangsfläche 8 übergeht, auf der ein Gewebeclip 9 (in aufgespanntem Zustand) axialbeweglich aufgelagert ist.

An dieser Stelle sei darauf hingewiesen, dass diese Außenumfangsfläche 8 die maximal radial äußere Fläche sein kann (wie in der Fig. 1 gezeigt) oder aber nochmals von einer radial weiter außen angeordneten Umfangsfläche unter Ausbildung eines axial sich erstreckenden Ringspalts zur Aufnahme des Gewebeclips 9 überragt/umgeben sein kann (in der Fig. 1 nicht dargestellt).

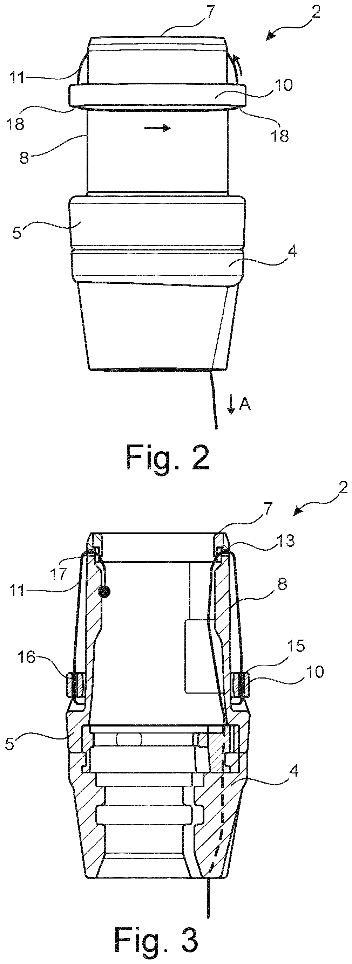

Wie in Fig. 1 zu erkennen, ist der Gewebeclip 9 an seiner proximalen Stirnseite/Rand mit einem ebenfalls auf der Außenumfangsfläche 8 des Gewebeclip-Halteabschnitts 5 abgestützten/gelagerten Abziehring 10 in Verbindung/Anlage. Der Abziehring 10 ist dabei so mit einem Abziehfaden 11 gekoppelt, dass, wie nachstehend näher beschrieben und in Fig. 2 gezeigt, bei manueller Betätigung des Abziehfadens 11 (Ziehen des Abziehfadens 11 in Richtung proximal) der Abziehring 10 in Richtung distal an dem Gewebeclip-Halteabschnitt 5 entlang gleitet und dabei den Gewebeclip 9 in Richtung distal verschiebt/ verlagert. Mit anderen Worten ausgedrückt, wird der Abziehring 10 und damit einhergehend auch der Gewebeclip 9 in axialer Richtung des Gewebeclip-Halteabschnitts 5 verlagert, wenn der Abziehfaden 11 gezogen wird.

Wie in Fig. 1 angedeutet, kann an dem Kappenaufsatz 2 eine Überwurfhülse 12 angeordnet/befestigt sein bzw. die Überwurfhülse 12 kann fest, insbesondere stoffeinstückig, mit dem Kappenaufsatz 2 verbunden sein. Diese Überwurfhülse 12 umgibt den auf den Gewebeclip-Halteabschnitt 5 aufgezogenen Gewebeclip 9 zumindest abschnittsweise und ermöglicht somit, eine zumindest teilweise Abschirmung der Abzieheinrichtung aus Abziehring 10 und Abziehfaden 11.

Fig. 3 zeigt einen Längsquerschnitt des Gewebeclip-Applikations-Ausrüst- oder Nachrüstsatzes 1 gemäß dem Ausführungsbeispiel.

In einem distalen (dem Benutzer abgewandten) Endbereich des Gewebeclip-Halteabschnitts 5 ist eine erste Radialdurchgangsöffnung 13 ausgebildet, welche den inneren Hohlraum 6 mit der Außenumfangsfläche 8 verbindet und durch welche der in dem Hohlraum 6 geführte Abziehfaden 11 geführt ist. Der Abziehfaden 11 erstreckt sich in dem Ausführungsbeispiel mit seinem proximalen Endabschnitt durch den Hohlraum 6 hin zu dem Endoskop 3 und wird durch einen separaten, an dem Endoskop 3, beispielsweise mit Clips und/oder Bändern, temporär angeordneten Längskanal 14 bis hin zu dem proximalen Endabschnitt des Endoskops 3, d.h. vorzugsweise aus dem Hohlorgan des Patienten, geführt, so dass der Abziehfaden 11 von außerhalb des Hohlorgans des Patienten, vorzugsweise manuell, bedient/betätigt werden kann. Alternativ kann der Abziehfaden 11 auch über den Hohlraum 6 in den Arbeitskanal des Endoskops 3 und dort bis zu dem proximalen Endabschnitt des Endoskops 3 geführt werden. Weiter alternativ ist es auch möglich, den Abziehfaden in einem separat zum Endoskop/Endoskopschaft gehaltenen Schlauch/Kanal zu führen, wodurch der Kappenaufsatz samt aufgezogenem Clip, der Schlauch sowie der darin längsgeführte Abziehfaden den einheitlichen Gewebeclip-Applikations-Ausrüst- oder Nachrüstsatz 1 bildet der quasi als wahlweise Smartfunktion dem (handelsüblichen) Endoskop beigegeben werden kann, ohne dass dessen interne Funktionen beeinträchtigt werden.

In dem Ausführungsbeispiel ist der Abziehfaden 11, wie vorstehend beschrieben, durch die erste Radialdurchgangsöffnung 13 ausgehend vom Hohlraum 6 radial auf die Außen-/Umfangsseite des Gewebeclip- Applikations-Ausrüst- oder Nachrüstsatz 1 geführt, von wo er in Richtung proximal durch eine in dem Abziehring 10 ausgebildete erste Axialdurchgangsöffnung 15 geführt ist. Anschließend läuft der Abziehfaden 10, wie in Fig. 2 gezeigt, entlang der Außenumfangsfläche 8 des (hülsenförmigen) Kappenaufsatzes 2 um den Gewebeclip-Halteabschnitt 5 (d.h. in Umfangsrichtung), bevor er durch eine in dem Abziehring 10, vorzugsweise um 180° versetzt zur ersten Axialdurchgangsöffnung 15, ausgebildete, zweite Axialdurchgangsöffnung 16 in Richtung distal zum distalen Endbereich des Gewebeclip-Halteabschnitts 5 geführt wird, wo er durch eine, vorzugsweise um 180° versetzt zur ersten Radialdurchgangsöffnung 13 ausgebildete, zweite Radialdurchgangsöffnung 17 in den Hohlraum 6 zurückgeführt wird, um (dort) an dem Gewebeclip-Halteabschnitt 5 befestigt zu werden.

Mit anderen Worten ausgedrückt, ist ein Ende des Abziehfadens 11 an dem proximalen Endabschnitt des Endoskops 3 angeordnet und das andere Ende des Abziehfadens 11, wie in Fig. 3 gezeigt, an dem Gewebeclip-Halteabschnitt 5 befestigt. Ausgehend von dem proximalen Endabschnitt des Endoskops 3 wird der Abziehfaden 11 durch den separaten Kanal oder den Arbeitskanal (Schlauch) oder durch einen Kanal des Endoskops 3 hin zu dessen distalen Endabschnitt und dort in den Hohlraum 6 des Gewebeclip-Applikations-Ausrüst- oder Nachrüstsatzes 1 geführt. An dem distalen Endbereich des Gewebeclip-Halteabschnitts 5 tritt der Abziehfaden 11 durch die erste Radialdurchgangsöffnung 13 auf die Außenseite, wo er in Richtung proximal entlang der Außenumfangsfläche 8, durch die erste Axialdurchgangsöffnung 15 des Abziehrings 10 und daraufhin entlang der Außenumfangsfläche 8 und durch die zweite Axialdurchgangsöffnung 16 des Abziehrings 10 wieder in Richtung distal geführt wird. Durch die an dem distalen Endbereich des Gewebeclip-Halteabschnitts 5 ausgebildete, zweite Radialdurchgangsöffnung 17 tritt der Abziehfaden 11 wieder in den Hohlraum 6 ein, um dort an dem Gewebeclip-Halteabschnitt 5 befestigt zu werden.

Diese Fadenführung entspricht im Wesentlichem einem einfachen Flaschenzug mit einem fest fixierten oberen Seilende, einem zunächst um eine obere Umlenkung geführten und anschließend nach unten reichenden (Betätigungs-)Ende sowie einem zwischen Fixierung und Umlenkung befindlichen Seilmittenabschnitt, der in eine Last eingeschlauft ist.

Wenn nun ein Benutzer den Abziehfaden 11 an dem proximalen Endabschnitt des Endoskops 3 betätigt, d.h. den Abziehfaden 11 in Richtung proximal zieht, wie durch den Pfeil A in Fig. 2 dargestellt, wirkt auf den Abziehring 10 zumindest an zwei Angriffspunkten 18 im Bereich der beiden Axialdurchgangsöffnungen 15, 16 eine Axialkraft in Richtung distal, welche den Abziehring 10 schließlich hin zu dem distalen Endbereich des Gewebeclip-Halteabschnitts 5 verschiebt/ verlagert, um den Gewebeclip 9 von dem Gewebeclip-Halteabschnitt 5 über dessen Vorderkante 7 abzustreifen. Da auf den Abziehring 10 zumindest an den beiden Angriffspunkten 18 jeweils eine Axialkraft wirkt, kann ein bei etwaiger Axialkrafteinleitung an nur einem Angriffspunkt auftretendes Kippmoment, welches den Abziehring 10 relativ zu seiner Mittelachse/ Rotationsachse kippt/ verdreht, beseitigt oder zumindest minimiert werden, wodurch wiederum das Risiko eines Verkantens des Abziehrings 10 an dem Gewebeclip-Halteabschnitt 5 verringert wird.

In anderen Worten ausgedrückt, kann die Funktion , welche nicht Teil der beanspruchten Erfindung ist, des Gewebeclip-Applikations-Ausrüst- oder Nachrüstsatzes 1 gemäß dem Ausführungsbeispiel bzw. der Modifikation des bevorzugten Ausführungsbeispiels im Allgemeinen wie folgt beschreiben werden: Zunächst wird der erfindungsgemäße Gewebeclip-Applikations-Ausrüst- oder Nachrüstsatz 1 auf den distalen Endabschnitt des (allgemein bekannten) Endoskops 3 aufgesetzt/ aufgestülpt. Über einen bekannten Ausrichtmechanismus, z.B. in Form von Bowdenzügen, kann der distale Endabschnitt des Endoskops 3 mitsamt darauf aufgestülptem Gewebeclip-Applikations-Ausrüst- oder Nachrüstsatz 1 zu einer Hohlorganwand weisend ausgerichtet werden. Sobald der Gewebeclip-Applikations-Ausrüst- oder Nachrüstsatz 1 distal stirnseitig, d.h. mit seiner distalen Vorderkante 7 (die ggf. abgerundet ist) gegen das Gewebe der Hohlorganwand des Patienten angepresst ist, kann das Patientengewebe in den Hohlraum 6 des Gewebeclip-Applikations-Ausrüst- oder Nachrüstsatz 1 eingezogen werden. Dies erfolgt mittels in dem Hohlraum 6 aufgebauten Unterdrucks und/oder mittels eines eingeführten Instruments (Zange, Haken, Gewebeanker, etc.). Zusätzlich kann über den inneren Arbeitskanal des Endoskops 3 ein weiteres medizinisches Instrument in den Hohlraum 6 eingeführt werden, um so das zu behandelnde Patientengewebe an zwei voneinander beabstandeten Stellen zu erfassen und in den Hohlraum 6 zu ziehen. Auf diese Weise kann das Patientengewebe besser gegriffen werden. Sobald ausreichend Gewebe in den Hohlraum 6 eingezogen ist, wird der Gewebeclip 9 mittels des Abziehfadens 11 in Richtung distal über die Vorderkante 7 abgestreift, wobei dieser hierauf das Patientengewebe zusammenklemmt.

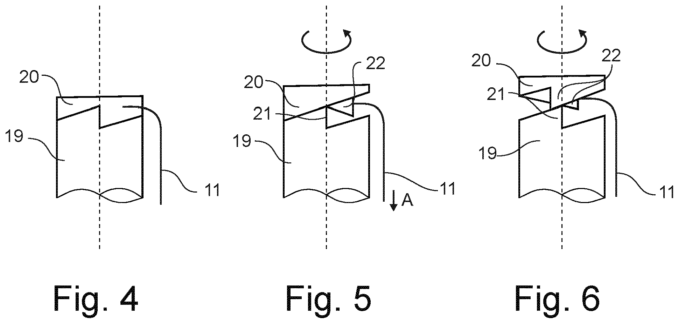

Die Fign. 4 bis 6 zeigen schematisch eine Abzieheinrichtung gemäß einem alternativen Ausführungsbeispiel, welches unabhängig vom vorstehenden Ausführungsbeispiel beansprucht werden kann, und nicht durch die beanspruchte Erfindung abgedeckt ist, wobei nachfolgend im Wesentlichen nur auf die zum ersten Ausführungsbeispiel unterschiedlichen Merkmale eingegangen werden soll.

Der Abziehring 10 ist demzufolge zweiteilig ausgeführt und weist einen ortsfesten, mit dem Kappenaufsatz 2 verbundenen Stützring/Stutzen 19 sowie einen relativ zu dem Stützring 19 verlagerbaren Schiebe-Dreh-Ring 20 (nachfolgend nur noch als Schiebering bezeichnet) auf. An einer Außenumfangsfläche des Schieberings 20 ist der Abziehfaden 11 befestigt, derart, dass dieser sich zumindest abschnittsweise in Umfangsrichtung erstreckt, so dass der Schiebering 20 bei Betätigung des Abziehfadens 11, d.h. wenn der Abziehfaden 11 in Richtung proximal gezogen wird, um seine Rotationsachse gedreht wird.

Wie in Fig. 4 zu erkennen ist, ist die dem Schiebering 20 zugewandte Stirnfläche des Stützrings 19 mit zwei Rampen/Nocken/Keilprofilen 21 ausgeführt. Zudem weist auch die dem Stützring 19 zugewandte Stirnfläche des Schieberings 20 zwei den Keilprofilen gegenläufigen Rampen 22 auf. D.h. die jeweiligen Stirnflächen des Stützrings 19 bzw. des Schieberings 20 sind mit Steigungsflächen/Profilen ausgebildet, derart, dass die Steigungsflächen/Profile, vorzugsweise die Rampen 21, 22 bei Rotation des Schieberings 20 relativ zum Stützring 19 aneinander abgleiten und so den Schiebering 20 und den Stützring 19 zunehmend axial voneinander beabstanden, wie in Fig. 5 und 6 angedeutet. Der Schiebering 20 drückt hierbei den Gewebeclip 9 in Richtung distal, um diesen von dem Gewebeclip-Halteabschnitt 5 abzustreifen.

In dem vorstehend beschriebenen Ausführungsbeispiel wird der Abziehfaden 11 zwischen der Außenumfangsfläche 8 und dem Gewebeclip 9 geführt. D.h. der Abziehfaden 11 verläuft zwischen der ersten Radialdurchgangsöffnung 13 und der ersten Axialdurchgangsöffnung 15 sowie zwischen der zweiten Axialdurchgangsöffnung 16 und der zweiten Radialdurchgangsöffnung 17 radial innen an dem Gewebeclip 9 vorbei. Dabei ist zudem denkbar, auf der Außenumfangsfläche 8 des Gewebeclip-Halteabschnitts 5 jeweils eine Axialnut auszubilden, in welcher der Abziehfaden 11 geführt wird, so dass der Gewebeclip 9 beim Abstreifen den in den Axialnuten geführten Abziehfaden 11 überstreicht.

Weiterhin ist in dem Ausführungsbeispiel ein Abziehfaden 11 so an dem Kappenaufsatz 2 entlanggeführt, dass sich bei Betätigung die zwei Angriffspunkte 18 einstellen.

Alternativ kann der Abziehfaden 11 jedoch auch so an dem Kappenaufsatz 2 entlanggeführt sein, dass sich mehr als zwei Angriffspunkte ausbilden. D.h. der Abziehfaden 11 wird durch weitere Radialdurchgangsöffnungen und Axialdurchgangsöffnungen geführt, um zusätzliche Angriffspunkte zu erhalten. Zwischen den Angriffspunkten muss dafür jedoch, wie bei einem Flaschenzug, jeweils distal eine Umlenkstelle angeordnet bzw. ausgebildet sein. Zur Ausbildung der Umlenkstelle kann der Abziehfaden 11 beispielsweise über die Vorderkante 7 nach innen in den Hohlraum 6, durch eine weitere, distale Radialdurchgangsöffnung wieder nach außen und dort zum nächsten proximalen Angriffspunkt am Abziehring 10 geführt werden.

Auch ist der Abziehfaden 11 in dem Ausführungsbeispiel innen im Bereich des Hohlraums 6 an dem Kappenaufsatz 2 fixiert. Jedoch ist auch eine andere Fixierungsstelle denkbar, solange der Abziehfaden 11 distal ortsfest gehalten ist. So kann der Abziehfaden 11 distal an der Außenumfangsfläche 8 fixiert sein oder an seinem distalen Ende einen Körper oder Knoten mit einer Querschnittsfläche größer als der Querschnittsfläche der zweiten Radialdurchgangsöffnung 17 aufweisen, welcher nach dem Durchtreten der zweiten Radialdurchgangsöffnung 17 ausgebildet oder angebracht wird, so dass das distale Ende des Abziehfadens 11 nicht mehr durch die zweite Radialdurchgangsöffnung 17 rutschen kann bzw. ortsfest gehalten wird.

Darüber hinaus ist der Abziehfaden 11 in dem Ausführungsbeispie an den Angriffspunkten durch die Axialdurchgangsöffnungen geführt. Jedoch ist auch denkbar, dass zur Umlenkung des Abziehfadens 11 an dem Abziehring 10 Ösen ausgebildet oder angeordnet sind.

Wie vorstehend erwähnt, stellen sich bei dem Ausführungsbeispie aufgrund der Führung des Abziehfadens 11 zwei Angriffspunkte 18 an dem Abziehring 10 ein. Jedoch kann alternativ zur Ausbildung der beiden Angriffspunkte auch ein zweiter Abziehfaden angeordnet werden bzw. eine Mehrzahl an Abziehfäden für eine Mehrzahl an Angriffspunkten. D.h. jedem Angriffspunkt kann ein separater Abziehfaden zugeordnet sein. Eine solche Ausführungsform ist jedoch nicht durch die beanspruchte Erfindung abgedeckt.

Ferner weisen der Stützring 19 und der Schiebering 20 in dem weiteren Ausführungsbeispiel die Rampen 21, 22 auf. Alternativ kann an dem Stützring 19 auch ein Innengewinde ausgebildet sein, welches mit einem an dem Schiebering 20 ausgebildeten Außengewinde in Eingriff steht, so dass bei einer Rotation des Schieberings 20 aufgrund der Betätigung des Abziehfadens 11 der Schiebering 20 relativ zu dem Stützring 19 in Richtung distal verlagert wird. Dabei ist es selbstverständlich auch denkbar, dass das Innengewinde an dem Schiebering 20 und das Außengewinde an dem Stützring 19 ausgebildet ist

### Bezugszeichenliste

- 1: Gewebeclip-Applikations-Ausrüst- oder Nachrüstsatz
- 2: Kappenaufsatz
- 3: Endoskop
- 4: Montage-/ Aufstülpabschnitt
- 5: Gewebeclip-Halteabschnitt
- 6: Hohlraum
- 7: Vorderkante
- 8: Mantelfläche/Außenumfangsfläche
- 9: Gewebeclip
- 10: Abziehring
- 11: Abziehfaden
- 12: Überwurfhülse
- 13: erste Radialdurchgangsöffnung
- 14: Längskanal
- 15: erste Axialdurchgangsöffnung
- 16: zweite Axialdurchgangsöffnung
- 17: zweite Radialdurchgangsöffnung
- 18: Angriffspunkt
- 19: Stützring
- 20: Schiebering
- 21: stützringseitige Rampe/Nocke
- 22: schieberingseitige Rampe/Nocke

## Patentansprüche

1. Gewebeclip-Applikations-Ausrüst- oder Nachrüstsatz (1) mit einem Kappenaufsatz (2), der dafür ausgebildet ist, an dem distalen Endabschnitt eines medizinischen Endoskops (3) der Schaftbauart montiert, insbesondere auf diesen aufgestülpt zu werden und der hierfür einen proximalen Montage- oder Aufstülpabschnitt(4) und einen distalen Gewebeclip-Halteabschnitt (5) aufweist, in dessen Bereich innenseitig ein zumindest in Richtung distal offener Hohlraum (6) ausgebildet ist und auf dessen einer, radial nach außen weisenden Umfangsfläche (8) ein Gewebeclip (9) radial abgestützt ist, welcher mittels einer Abzieheinrichtung in Form eines Seilzug-betätigbaren, auf der radialen Außenumfangsfläche (8) des Gewebeclip-Halteabschnitts (5) axialgleitend gelagerten und den Gewebeclip (9) verschiebenden Abziehrings (10) in Richtung distal verlagerbar ist, wobei der Seilzug direkt mit dem Abziehring (10) an wenigstens zwei Anlenk- oder Angriffspunkten (18) in Schiebekraft-Einleitungskontakt ist, wobei die zumindest zwei Anlenk- oder Angriffspunkte (18) gleichmäßig, vorzugsweise sich radial gegenüberliegend verteilt sind, **dadurch gekennzeichnet, dass** der Seilzug ein Abziehfaden (10) ist oder hat, der an seinem einen Ende am Kappenaufsatz (2) fixiert ist, hiervon ausgehend an einem der zwei Angriffspunkte (18) mit dem Abziehring (10) längsverschiebbar in Eingriff gebracht ist, von diesem einen Angriffspunkt ausgehend an einem anderen der zwei Angriffspunkte (18) mit dem Abziehring (10) längsverschiebbar in Eingriff gebracht ist, von diesem anderen Angriffspunkt ausgehend um einen distalen Endabschnitt des Kappenaufsatzes (2) herum, vorzugsweise in dessen Hohlraum (5), in Richtung proximal umgeleitet und von dort zu einem proximalen Endabschnitt des Endoskops (3) geführt ist.

2. Gewebeclip-Applikations-Ausrüst- oder Nachrüstsatz (1) nach Anspruch 1, **dadurch gekennzeichnet, dass** der Abziehfaden (11) zwischen dem einen und dem anderen der zwei Angriffspunkte (18) so an der radial nach außen weisenden Umfangsfläche (8) des Kappenaufsatzes (2) gleitend längsgeführt ist, dass bei einer Betätigung des Abziehfadens (11) dieser den Abziehring (10) an den zumindest zwei Angriffspunkten (18) flaschenzugartig in Richtung distal zieht.

3. Gewebeclip-Applikations-Ausrüst- oder Nachrüstsatz (1) nach Anspruch 2, **dadurch gekennzeichnet, dass** der Abziehfaden (11) im Umfangsabschnitt zwischen den zwei Angriffspunkten (18) an der proximalen Stirnseite des Abziehrings (10) gleitend anliegt.

4. Gewebeclip-Applikations-Ausrüst- oder Nachrüstsatz (1) nach Anspruch 1, **dadurch gekennzeichnet, dass** der Abziehfaden (11) von dem Hohlraum (6) über eine in dem Gewebeclip-Halteabschnitt (5) ausgebildete, erste distale Radialdurchgangsöffnung (13) nach radial außen, durch eine in dem Abziehring (10) ausgebildete, erste Axialdurchgangsöffnung (15) entlang der Außenumfangsfläche (8) und durch eine in dem Abziehring (10), vorzugsweise um 180° versetzt zu der ersten Axialdurchgangsöffnung (15), ausgebildete, zweite Axialdurchgangsöffnung (16) sowie eine in dem Gewebeclip-Halteabschnitt (5), vorzugsweise um 180° versetzt zu der ersten Radialdurchgangsöffnung (13), ausgebildete, zweite distale Radialdurchgangsöffnung (17) geführt ist.

5. Gewebeclip-Applikations-Ausrüst- oder Nachrüstsatz (1) nach Anspruch 1, **dadurch gekennzeichnet, dass** an dem Abziehring (10) zumindest zwei, den Angriffspunkten (18) zugeordnete Abziehfäden angeordnet sind.

## Claims

1. A tissue clip application fitting or retrofitting set (1) comprising a cap attachment (2) which is designed to be mounted on the distal end portion of a shaft-type medical endoscope (3), in particular to be placed thereon, and which for this purpose has a proximal mounting or placement portion (4) and a distal tissue clip holding portion (5), in the region of which a cavity (6) open at least in the distal direction is formed on the inside and on a radially outward-pointing peripheral surface (8) of which a tissue clip (9) is radially supported and can be displaced in the distal direction by means of a pull-off device in the form of a pull-off ring (10) which can be cable-pull operated, is mounted on the radial outer peripheral surface (8) of the tissue clip holding portion (5) so that it can axially slide and move the tissue clip (9), wherein the cable pull is in sliding force initiation contact with the pull-off ring (10) in at least two coupling or engagement points (18), the at least two coupling or engagement points (18) being evenly distributed, preferably so as to be radially opposite to one another, **characterized in**
**that** the cable pull is or has a pull-off thread (10) which is fixed at one end to the cap attachment (2), is brought into longitudinally displaceable engagement therefrom with the pull-off ring (10) at one of the two engagement points (18), is brought, from another one of the two engagement points (18), into longitudinally displaceable engagement with the pull-off ring (10), is diverted from this other engagement point around a distal end portion of the cap attachment (2), preferably in the cavity (5) thereof, in a proximal direction and is guided from there to a proximal end portion of the endoscope (3).

2. The tissue clip application fitting or retrofitting set (1) according to claim 1, **characterized in that** the pull-off thread (11) between the one and the other one of the two engagement points (18) is guided in a sliding manner on the radially outward-pointing peripheral surface (8) of the cap attachment (2) such that in actuating the pull-off thread (11), the pull-off thread (11) pulls the pull-off ring (10) in a distal direction in the at least two engagement points (18) in the manner of a pulley.

3. The tissue clip application fitting or retrofitting set (1) according to claim 2, **characterized in that** the pull-off thread (11), in the peripheral section between the two engagement points (18), is in sliding contact with the proximal front side of the pull-off ring (10).

4. The tissue clip application fitting or retrofitting set (1) according to claim 1, **characterized in that** the pull-off thread (11) is guided from the cavity (6) via a first distal radial through opening (13) formed in the tissue clip holding portion (5) radially outwardly, through a first axial through opening (15) formed in the pull-off ring (10) along the outer peripheral surface (8) and through a second axial through opening (16) formed in the pull-off ring (10), preferably displaced by 180° with respect to the first axial through opening (15), and a second distal radial through opening (17) formed in the tissue clip holding portion (5) and preferably displaced by 180° with respect to the first radial through opening (13).

5. The tissue clip application fitting or retrofitting set (1) according to claim 2, **characterized in that** on the pull-off ring (10) at least two pull-off threads are arranged that are associated with the engagement points (18).

## Revendications

1. Kit d'équipement (1) ou kit d'équipement ultérieur pour application de clips tissulaires, comprenant un embout de type capuchon (2) qui est conçu pour être monté, en particulier mis en place, sur une partie terminale distale d'un endoscope médical (3) du type tige, et comprenant à cet effet une partie de montage ou de mise en place proximale (4) et une partie de retenue de clip tissulaire distale (5), dans la région de laquelle une cavité (6) ouverte au moins dans la direction distale est formée côté intérieur, un clip tissulaire (9) étant mis en place radialement sur sa surface périphérique (8) orientée radialement vers l'extérieur, qui peut être déplacé dans la direction distale au moyen d'un dispositif de retrait qui se présente sous la forme d'une bague de traction (10) pouvant être actionnée par un câble, est monté sur la surface périphérique extérieure radiale (8) de la partie de retenue de clip tissulaire (5) de manière à pouvoir coulisser axialement et qui déplace le clip tissulaire (9), dans lequel le câble se trouve en contact directement avec la bague de traction (10) au niveau d'au moins deux points d'articulation ou de mise en prise (18), dans lequel les au moins deux points d'articulation ou de mise en prise (18) sont répartis régulièrement, de préférence radialement opposés de manière à permettre l'application d'une force de coulissement l'un à l'autre, **caractérisé en ce que** le câble de traction est ou présente un fil de traction (10) qui est fixé à son extrémité à l'embout de type capuchon (2) et, à partir de là, est mis en prise de manière déplaçable longitudinalement avec la bague de traction (10) au niveau d'un premier des deux points de mise en prise (18), à partir de ce premier point de mise en prise est mis en prise de manière déplaçable longitudinalement avec la bague de traction (10) au niveau d'un autre des deux points de mise en prise (18), à partir de cet autre point de mise en prise est redirigé autour d'un tronçon d'extrémité distale de l'embout de type capuchon (2), de préférence dans sa cavité (5), dans une direction proximale et guidé de là vers une partie d'extrémité proximale de l'endoscope (3).

2. Kit d'équipement (1) ou kit d'équipement ultérieur pour application de clips tissulaires selon la revendication 1, **caractérisé en ce que le** fil de traction (11) est guidé longitudinalement de manière coulissante entre le premier et l'autre des deux points de mise en prise (18) sur la surface périphérique (8) dirigée radialement vers l'extérieur de l'embout de type capuchon (2), de sorte que lorsque le fil de traction (11) est actionné, il tire la bague de traction (10) au niveau des au moins deux points de mise en prise (18) comme un palan dans une direction distale.

3. Kit d'équipement (1) ou kit d'équipement ultérieur pour application de clips tissulaires selon la revendication 2, **caractérisé en ce que** le fil de traction (11) coulisse dans la partie périphérique entre les deux points de mise en prise (18) sur la face d'extrémité proximale de la bague de traction (10).

4. Kit d'équipement (1) ou un kit d'équipement ultérieur pour application de clips tissulaires selon la revendication 1, **caractérisé en ce que** le fil de traction (11) est guidé depuis la cavité (6) radialement vers l'extérieur via une première ouverture traversante radiale distale (13) formée dans la partie de retenue de clip tissulaire (5), à travers une première ouverture traversante axiale (15) formée dans la bague de traction (10) le long de la surface périphérique extérieure (8) et à travers une seconde ouverture traversante axiale (16) formée dans la bague de traction (10), de préférence formée décalée de 180° par rapport à la première ouverture traversante axiale (15), et une seconde ouverture traversante radiale distale (17) dans la partie de retenue de clip tissulaire (5), de préférence formée décalée de 180° par rapport à la première ouverture de passage radiale (13).

5. Kit d'équipement (1) ou kit d'équipement ultérieur pour application de clips tissulaires selon la revendication 1, **caractérisé en ce qu'**au moins deux fils de traction associés aux points de mise en prise (18) sont agencés au niveau de la bague de traction (10).
